# FASCICULE DE BREVET EUROPEEN

(11) **EP 2 456 854 B1**
(45) Date de publication et mention de la délivrance du brevet: **30.08.2017**
(21) Numéro de dépôt: 10726999.5
(22) Date de dépôt: 02.07.2010
(51) Int. Cl.: C12N 5/077

(54) **PROCEDE D'OBTENTION DE MYOFIBROBLASTES**
VERFAHREN ZUR GEWINNUNG VON MYOFIBROBLASTEN
METHOD FOR OBTAINING MYOFIBROBLASTS

(30) Priorité: 24.07.2009 FR 0955216
(43) Date de publication de la demande: 30.05.2012
(73) Titulaire: Oncobiotek, 06390 Chateauneuf-Villevieille (FR)
(72) Inventeur: ROUYER, Nicolas, Jacques, F-06330 Roquefort Les Pins (FR); BARTHEL, Robert, F-06390 Chateauneuf-Villevieille (FR)
(74) Mandataire: Novagraaf Technologies
(86) Numéro de dépôt international: PCT/EP2010/059478
(87) Numéro de publication internationale: WO 2011/009706

(56) Documents cités:
- EP-A1- 1 715 033
- WEBBER MUKTA M ET AL: "A human prostatic stromal myofibroblast cell line WPMY-1: A model for stromal-epithelial interactions in prostatic neoplasia" CARCINOGENESIS (OXFORD), vol. 20, no. 7, juillet 1999 (1999-07), pages 1185-1192, XP002562726 ISSN: 0143-3334
- JESTER J V ET AL: "Induction of alpha-smooth muscle actin expression and myofibroblast transformation in cultured corneal keratocytes." CORNEA SEP 1996, vol. 15, no. 5, septembre 1996 (1996-09), pages 505-516, XP009127731 ISSN: 0277-3740
- MICERA A ET AL: "Nerve Growth Factor (NGF) Activates Conjunctival Myofibroblasts: a Role for NGF in Healing Processes and Tissue Remodeling." ARVO ANNUAL MEETING ABSTRACT SEARCH AND PROGRAM PLANNER, vol. 2002, 2002, XP002562727 & ANNUAL MEETING OF THE ASSOCIATION FOR RESEARCH IN VISION AND OPHTHALMOLOGY; FORT LAUDERDALE, FLORIDA, USA; MAY 05-10, 2002
- KHOUW ILSE M S L ET AL: "TGF-beta and bFGF affect the differentiation of proliferating porcine fibroblasts into myofibroblasts in vitro" BIOMATERIALS, vol. 20, no. 19, octobre 1999 (1999-10), pages 1815-1822, XP002562728 ISSN: 0142-9612
- GRUPP CLEMENS ET AL: "A novel model to study renal myofibroblast formation in vitro" KIDNEY INTERNATIONAL, vol. 59, no. 2, février 2001 (2001-02), pages 543-553, XP002562729 ISSN: 0085-2538

## Description

La présente invention concerne un procédé d'obtention de myofibroblastes caractérisé en ce que on met en culture un échantillon de cellules issu d'une tumeur, de préférence un carcinome dans un milieu de culture sans sérum, ledit milieu étant le MEGM comprenant de l'insuline, de l'hydrocortisone, de l'EGF, un extrait hypophysaire bovin et un antibiotique..

Dans la description ci-dessous, les références entre crochets ([[]) renvoient à la liste des références présentée à la fin du texte.

Les myofibroblastes représentent un type particulier de fibroblastes appelés CAFs, pour l'anglais « *carcinoma-associated fibroblasts* », autrement dit fibroblastes associés à un carcinome. Ils sont impliqués également dans une variété de processus biologiques tels que par exemple le remodelage tissulaire.

Ils expriment le marqueur alpha-actine de muscle lisse ou SMA.

Dans ce qui suit, le terme fibroblaste est utilisé au sens large. Il regroupe non seulement les fibroblastes, appelés ici fibroblastes « au sens strict », mais aussi les dérivés de fibroblastes tels que les fibroblastes activés, les CAFs, les myofibroblastes. Ainsi, on inclut les fibroblastes impliqués dans les processus tumoraux mais aussi ceux impliqués dans tout autre processus biologique dans lequel interviennent des myofibroblastes.

Il est connu notamment d'obtenir des fibroblastes à partir d'une tumeur, par digestion enzymatique de la tumeur suivie ou non d'une ou plusieurs étapes de purification (Orimo et al., Cell 2005, 121: 335-348 [1] ou Allinen et al., Cancer Cell 2004, 6:17-32 [2]).

Jusqu'à présent, les cultures de fibroblastes sont réalisées dans un milieu qui contient du sérum, classiquement de 5 à 20% de sérum, et contiennent un pourcentage variable de myofibroblastes.

Par exemple, dans le commerce, on trouve des CAFs dans un milieu à 5% de sérum (Asterand), qui sont déterminés comme tels par simple inspection visuelle.

Des laboratoires académiques produisent des populations de CAFs en milieu de culture à 10% de sérum. Le phénotype de ces cellules est déterminé à l'aide de la mesure du marqueur alpha-actine de muscle lisse. Ainsi, ces populations contiennent un pourcentage variable de CAFs, en moyenne au plus 30%.

On trouve aussi dans le commerce des myofibroblastes hépatiques humains (Dominion Pharmakine) dans un milieu à 20% de sérum, dont seulement 50% à 60% expriment le marqueur alpha-actine de muscle lisse (SMA). Dans le foie ainsi que dans le pancréas, la cellule à l'origine du myofibroblaste est la cellule étoilée, « *stellate cell* » en anglais, qui a pour particularité de présenter une activation spontanée avec expression du marqueur SMA lorsqu'elle est mise en culture (Kinnman et al., Lab Invest 2001, 81:1709-1716 [3]; Omary et al., JCI 2007, 117:50-59 [4]).

L'utilisation de sérum, par exemple de veau foetal ou de nouveau-né, présente pourtant les inconvénients suivants :
- problème éthique lié à la collecte du sérum,
- le sérum est un liquide biologique dont la composition est variable,
- la composition exacte du sérum n'est pas connue et il n'est pas possible de définir précisément les besoins de la cellule,
- le sérum contient des facteurs (facteurs de croissance, antagonistes...) qui peuvent interférer avec un test utilisé dans l'industrie pharmaceutique sur les cellules en culture,
- les cellules qui ont l'habitude de pousser en sérum changent de phénotype ou meurent lorsqu'on supprime le sérum dans le milieu de culture.

En résumé, les populations de fibroblastes, isolées à partir de tissus pathologiques, telles que connues et étudiées in vitro à ce jour:
- sont hétérogènes,
- contiennent un pourcentage variable de myofibroblastes, et
- poussent dans un milieu qui contient du sérum, dont la présence a les inconvénients mentionnés ci-dessus.

Dans ce contexte, le but principal visé par l'invention est d'obtenir une population de myofibroblastes, dont les caractéristiques facilitent toute étude de ces cellules, et en particulier une population la plus pure possible en myofibroblastes.

La présente invention a pour objet un procédé d'obtention de myofibroblastes, caractérisé en ce que on met en culture un échantillon de cellules issu d'une tumeur, de préférence un carcinome dans un milieu de culture sans sérum, ledit milieu étant le MEGM comprenant de l'insuline, de l'hydrocortisone, de l'EGF, un extrait hypophysaire bovin et un antibiotique.

Un procédé d'obtention de myofibroblastes, caractérisé en ce que :
(a) on prépare un échantillon de cellules incluant essentiellement des fibroblastes; et
(b) on met en culture cet échantillon de cellules dans un milieu de culture sans sérum est décrit.

L'échantillon de cellules incluant essentiellement des fibroblastes signifie qu'au moins 50% des cellules qu'il contient sont des fibroblastes, de préférence au moins 70%. Toutefois, l'homme du métier saura évaluer, par des expériences de routine, le pourcentage de fibroblastes que doit contenir l'échantillon de cellules pour la réalisation.

L'invention présente l'avantage de produire une population de myofibroblastes, plus pure que celles de l'art antérieur, et qui poussent dans un milieu qui ne contient pas de sérum.

Ainsi, les myofibroblastes obtenus par le procédé selon l'invention permettent par exemple d'étudier avec précision et de manière reproductible la biologie de ces cellules, et d'identifier des cibles thérapeutiques potentielles.

Du fait de l'absence de sérum, ils permettent aussi d'étudier les besoins des cellules et de définir les facteurs qui, par exemple, induisent leur différenciation, leur permettent de maintenir leur phénotype, soutiennent leur multiplication et/ou sont impliqués dans la sénescence prématurée de ces cellules.

Au bout de un ou deux passages en milieu sans sérum, la population de myofibroblastes obtenus avec le procédé selon l'invention peut aller jusqu'à plus de 95% de myofibroblastes.

Le milieu de culture sans sérum comprend de préférence un milieu de base sans sérum qui est un milieu de culture de cellules épithéliales. En effet, le milieu de culture obtenu à partir d'un tel milieu de base permet d'obtenir une très bonne pousse des myofibroblastes.

Ce milieu de base sans sérum est un milieu de culture de cellules épithéliales mammaires humaines. A titre d'exemple, ce peut être autrement un milieu de culture sans sérum connu pour la culture de cellules épithéliales bronchiques, placentaires ou rénales.

Ce milieu de base sans sérum est supplémenté à l'aide d'un ou plusieurs suppléments classiquement utilisés en culture cellulaire. A titre d'exemple de suppléments, on peut citer les hormones, les facteurs de croissance, les mitogènes, les antibiotiques. Il comprend de l'insuline, l'hydrocortisone, le facteur de croissance épidermique ou EGF, un extrait hypophysaire bovin ou BPE, et un antibiotique.

L'antibiotique n'est pas essentiel pour la bonne pousse des cellules; mais sa présence prévient la contamination microbienne.

L'antibiotique peut être par exemple le GA-1000 incluant la gentamicine et l'amphotéricine-B, ou la normocine. Tout antibiotique utilisé en routine en culture cellulaire peut être utilisé dans l'invention, et, à titre d'exemple, on peut citer la penicilline, la streptomycine, l'ampicilline, la kanamycine, la tylosine.

Le milieu de culture sans sérum selon l'invention est le milieu de culture de cellules épithéliales mammaires humaines tel que le milieu trouvé dans le commerce sous le nom de MEGM, pour l'anglais « *Mammary Epithelial Growth Medium* » (Cambrex) comprenant de l'insuline, de l'hydrocortisone, de l'EGF, un extrait hypophysaire bovin et un antibiotique.

A titre d'exemple un milieu de culture sans sérum connu pour la culture de cellules épithéliales bronchiques, placentaires ou rénales est décrit.

La formulation initiale du MEGM est celle du milieu MCDB 170 (Hammond et al., PNAS 1984, 81: 5435-5439 [5]). Ce milieu MCDB 170, puis MEGM, a été développé et utilisé jusqu'ici spécifiquement pour la culture de cellules épithéliales mammaires humaines normales.

Dans la présente invention, l'utilisation de ce milieu est particulièrement avantageuse pour la culture de myofibroblastes.

Il se compose d'un milieu de base, dont la formulation exacte est connue sous le nom de milieu MEBM, pour l'anglais *« Mammary Epithelial Basal Medium* », et de cinq suppléments: insuline, hydrocortisone, EGF, extrait hypophysaire bovin et un antibiotique, le GA-1000.

L'éthanolamine et la phosphoéthanolamine sont des précurseurs lipidiques inclus dans la formulation de base du MCDB 170 et dans le MEGM.

Un autre exemple de milieu de base sans sérum est présenté dans le tableau 1. La colonne de gauche liste les produits entrant dans la composition de ce milieu. La colonne de droite indique, pour chaque produit, sa concentration molaire dans le milieu, en moles par litre de milieu.

Ce milieu de base sans sérum comprend en fait un mélange des milieux de culture DMEM et HamF12 (Invitrogen) en proportions 1:1.

Ce milieu de base, mélangé à de l'EGF (Peprotech), de l'hydrocortisone (SIGMA), du BPE (Invitrogen), de l'ITS-X (Invitrogen) qui inclut insuline, transferrine et sélénium, et un antiobiotique tel que la normocine (Invivogen), donne un milieu de culture sans sérum qui peut être utilisé dans l'invention. On appelle le milieu de culture obtenu « milieu de culture sans sérum DMEM/HamF12 ».

Les compositions du DMEM et du HamF12 sont celles indiquées par exemple dans R. Ian Freshney, « *Culture of Animal Cells A Manual of Basic Technique* », 2005 [6].

**Tableau 1**

| **Composante** | **Concentration molaire** |
|---|---|
| L-alanine | 5X10⁻⁵ |
| L-arginine | 7X10⁻⁴ |
| L-asparagine | 5X10⁻⁵ |
| L-acide aspartique | 5X10⁻⁵ |
| L-cystéine | 10⁻⁴ |
| L-cystine | 10⁻⁴ |
| L-acide glutamique | 5X10⁻⁵ |
| L-glutamine | 2,5X10⁻³ |
| Glycine | 2,5X10⁻⁴ |
| L-histidine | 1,5X10⁻⁴ |
| L-isoleucine | 4,2X10⁻⁴ |
| L-leucine | 4,5X10⁻⁴ |
| L-lysine HCl | 5X10⁻⁴ |
| L-méthionine | 1,2X10⁻⁴ |
| L-phénylalanine | 2,2X10⁻⁴ |
| L-proline | 1,5X10⁻⁴ |
| L-sérine | 2,5X10⁻⁴ |
| L-thréonine | 4,5X10⁻⁴ |
| L-tryptophane | 4,4X10⁻⁵ |
| L-tyrosine | 2,1X10⁻⁴ |
| L-valine | 4,5X10⁻⁴ |
| Biotine | 1,5X10⁻⁸ |
| Chlorure de choline | 6,4X10⁻⁵ |
| Acide folique | 6X10⁻⁶ |
| Myo-inositol | 7X10⁻⁵ |
| Nicotinamide | 1,7X10⁻⁵ |
| Pantothénate D-Ca | 9,4X10⁻⁶ |
| Pyridoxal HCl | 10⁻⁵ |
| Pyridoxine HCl | 1,5X10⁻⁷ |
| Riboflavine | 5,8X10⁻⁷ |
| Thiamine | 6,4X10⁻⁶ |
| Vitamine B12 | 5X10⁻⁷ |
| CaCl₂ | 1,1X10⁻³ |
| KCl | 4,2X10⁻³ |
| MgSO₄ | 4X10⁻⁴ |
| NaCl | 1,2X10⁻¹ |
| NaHCO₃ | 2,9X10⁻² |
| NaH₂P₄ | 4,5X10⁻⁴ |
| Na₂HPO₄ | 5X10⁻⁴ |
| CuSO₄ 5H₂O | 7,8X10⁻⁹ |
| Fe(NO₃)₃ 9H₂O | 1,2X10⁻⁷ |
| FeSO₄ 7H₂O | 1,5X10⁻⁶ |
| ZnSO₄ 7H₂O | 1,5X10⁻⁶ |
| Hypoxanthine | 1,5X10⁻⁵ |
| Thymidine | 1,5X10⁻⁶ |
| D-glucose | 1,8X10⁻² |
| Pyruvate de sodium | 10⁻³ |
| Acide linoléique | 1,5X10⁻⁷ |
| Acide lipoïque | 5,1X10⁻⁷ |
| Rouge phénol | 3,6X10⁻⁵ |
| Putrescine | 5X10⁻⁷ |

Selon un mode de réalisation de l'invention, l'étape (a) comprend une obtention d'une suspension cellulaire à partir d'un échantillon biologique tel qu'un tissu biologique; puis une culture initiale des cellules obtenues dans un milieu de culture favorisant la pousse de fibroblastes, par exemple dans un milieu de culture contenant du sérum.

Selon un autre mode de réalisation de l'invention, l'étape (a) comprend une obtention d'une suspension cellulaire à partir d'un échantillon biologique tel qu'un tissu biologique; puis une purification de sous-populations cellulaires pour obtenir l'échantillon de cellules incluant essentiellement des fibroblastes.

L'obtention d'une suspension cellulaire à partir d'un échantillon biologique peut se faire par digestion enzymatique ou par toute autre méthode telle que la dissociation mécanique ou les tamis cellulaires.

On préfère la digestion enzymatique parce que cette méthode est simple et efficace.

L'échantillon biologique peut être de toute origine lui permettant d'inclure essentiellement des fibroblastes.

Ainsi, il peut venir de toute espèce de mammifère.

Notamment, l'échantillon biologique est issu d'une tumeur, de préférence un carcinome. Tout autre tissu pathologique tel que tout tissu en remodelage est décrit.

Pour l'invention, l'essentiel est que l'échantillon biologique contienne des fibroblastes et en particulier des myofibroblastes.

Le pourcentage de myofibroblastes dans l'échantillon biologique est évalué sur une coupe histologique avec un marquage immunohistochimique pour l'alpha-actine de muscle lisse.

A titre d'exemple, il est préférable de partir d'un échantillon biologique dont au moins 30%, ou plus préférentiellement au moins 50%, des fibroblastes ont le phénotype myofibroblaste.

Le matériel de départ pour la culture peut être n'importe quel tissu de mammifère qui contient des myofibroblastes : une tumeur, par exemple un carcinome. Un tissu faisant l'objet de remodelage ou de réparation tissulaire, tel qu'en cas de fibrose, cirrhose, cicatrice ou plaie est décrit. Les myofibroblastes jouent en effet un rôle clé dans tous ces processus.

Le milieu de culture favorisant la pousse de fibroblastes est plus classiquement un milieu contenant du sérum. Il peut s'agir par exemple d'un milieu de culture à base de RPMI, de DMEM ou de HAMF12.

Pour purifier des sous-populations cellulaires afin d'obtenir l'échantillon de cellules incluant essentiellement des fibroblastes, on peut par exemple mettre en oeuvre le protocole décrit par Allinen et al [2] ou celui décrit par Orimo et al [1].

Dans l'invention, il est possible de partir d'un mélange complexe de cellules épithéliales et stromales, sans purification préalable. Toutefois, une culture initiale dans un milieu contenant du sérum permet d'enrichir la culture en cellules fibroblastiques.

L'invention concerne également une culture cellulaire de myofibroblastes obtenue par le procédé selon l'invention, caractérisée en ce qu'au moins 80%, ou préférentiellement au moins 95%, des cellules qu'elle contient sont des myofibroblastes.

Cette culture cellulaire selon l'invention est de manière avantageuse dépourvue de sérum.

Une telle culture cellulaire permet d'étudier les effets d'une stimulation de l'activité des cellules, comme par exemple en cas de plaies qui ne guérissent pas, ou de déficit d'angiogenèse, ou au contraire les effets d'une inhibition de l'activité des cellules, comme notamment en cas de cancer, fibrose, ou cirrhose.

L'invention concerne aussi l'utilisation du procédé selon l'invention, pour obtenir une culture de cellules dans laquelle les cellules incluent au moins 80%, ou préférentiellement au moins 95%, de myofibroblastes.

Cette culture de cellules est de manière avantageuse dans un milieu sans sérum ledit milieu étant le MEGM comprenant de l'insuline, de l'hydrocortisone, de l'EGF, un extrait hypophysaire bovin et un antibiotique.

L'utilisation d'un milieu de culture sans sérum, développé pour la culture de cellules épithéliales mammaires humaines, pour l'obtention de myofibroblastes à partir d'un échantillon de cellules incluant essentiellement des fibroblastes est décrite.

Ce milieu de culture sans sérum peut comprendre au moins un supplément choisi par exemple parmi l'insuline, l'hydrocortisone, l'EGF, un extrait hypophysaire bovin et un antibiotique.

Dans l'invention, les cultures de cellules peuvent se faire à l'aide de tout moyen approprié, en suspension ou sur un support, en boîte ou en flasque, etc. L'homme du métier est en mesure de choisir ces moyens en faisant appel à ses connaissances générales.

Ainsi, quelques exemples d'application de l'invention sont: identification de biomarqueurs de myofibroblastes, identification de cibles thérapeutiques, identification et validation de composés anticancéreux, modèle in vitro pour le criblage de composés pharmaceutiques ou cosmétiques, toxicologie in vitro.

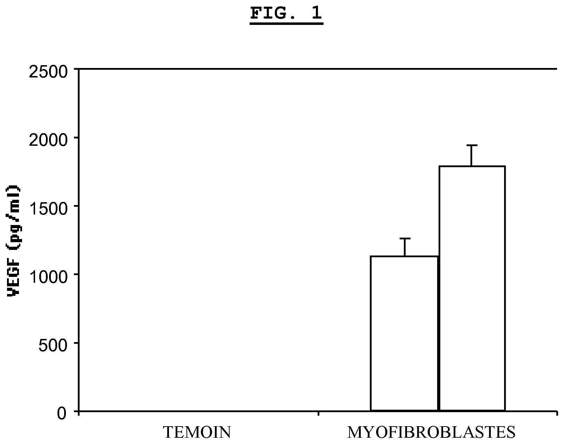

D'autres caractéristiques et avantages de l'invention ressortiront clairement de la description détaillée qui en est faite ci-après, à titre indicatif et nullement limitatif, en référence à la figure 1, qui représente un diagramme en colonnes de la quantité de VEGF produit par les myofibroblastes obtenus selon l'invention, en picogrammes par millilitre de milieu.

### Exemple de procédé d'obtention de myofibroblastes selon l'invention :

On part d'une tumeur prélevée chez un mammifère, dans le cas présent un carcinome prélevé sur un chien.

A titre d'exemple, une variante serait de partir d'un foie cirrhotique pour obtenir une population de myofibroblastes impliqués dans ce processus pathologique.

On peut réaliser un examen microscopique de la tumeur pour évaluer le pourcentage de myofibroblastes dans la tumeur, par exemple à l'aide d'un marquage immunohistochimique du tissu préalablement fixé en formol.

Idéalement, on part d'une tumeur qui contient un pourcentage élevé de myofibroblastes, c'est-à-dire de préférence au moins 30% des fibroblastes sont des myofibroblastes.

On réalise une digestion enzymatique de la tumeur suivie ou non d'une purification de sous-populations cellulaires.

Pour la digestion enzymatique, qui peut être remplacée par tout autre procédé permettant d'obtenir une suspension cellulaire, on peut procéder de trois façons différentes :
1) on conserve d'abord le tissu à 4°C pendant l'examen microscopique de la tumeur, en attendant les résultats de cet examen ; un inconvénient est que le tissu est susceptible de se dégrader.
2) on procède à la digestion enzymatique du tissu, avec ou sans purification de sous-populations cellulaires, puis on congèle les cellules en attendant le résultat de l'examen microscopique.
3) on procède à la digestion enzymatique du tissu et on initie la culture le jour même.

Après la digestion enzymatique, on peut ne pas conduire de purification de sous-populations cellulaires. Dans ce cas, on démarre la culture dans un milieu pour favoriser la pousse des fibroblastes. Au premier passage, on transfère les cellules dans le milieu sans sérum MEGM.

Après la digestion enzymatique, on peut autrement réaliser une purification de sous-populations cellulaires. Dans ce cas, après cette purification, on fait pousser directement la fraction contenant les fibroblastes et dérivés dans le milieu sans sérum MEGM.

A chaque passage, on peut réaliser une immunocytochimie pour déterminer le pourcentage de cellules positives au marqueur SMA.

Le milieu MEGM a été développé spécifiquement pour la culture de cellules épithéliales mammaires humaines normales.

Au bout de un ou deux passages, on obtient une culture de myofibroblastes qui sont, à plus de 95% et jusqu'à 100%, positifs au marqueur SMA.

Les cellules ainsi obtenues présentent notamment les avantages de maintenir leur phénotype en culture et de pousser de manière active sur environ 4 passages. Au cinquième passage seulement, elles montrent des signes de mort cellulaire ou sénescence.

En suivant le même procédé que dans l'exemple ci-dessus, à la seule différence que le milieu de culture MEGM est remplacé par le milieu de culture sans sérum DMEM/HamF12 décrit plus haut, on obtient des résultats similaires à ceux obtenus avec le milieu de culture MEGM : au bout de un ou deux passages, on obtient une culture de myofibroblastes qui sont, à plus de 95% et jusqu'à 100%, positifs au marqueur SMA. Toutefois, avec ce milieu de culture sans sérum DMEM/HamF12, les cellules poussent moins bien dès le quatrième passage.

### Exemple de protocole expérimental à suivre pour la mise en oeuvre du procédé d'obtention de myofibroblastes selon l'invention :

On part d'une tumeur mammaire, conservée par exemple dans un milieu de transport adapté.

On rince le prélèvement dans un tampon phosphate salin ou PBS.

On transfère des morceaux de tissu dans une boîte de culture contenant un cocktail enzymatique : collagénase et hyaluronidase dans un milieu de culture DMEM.

On dilacère le tissu en petits fragments à l'aide de deux scalpels, et on mélange intimement avec une pipette.

On place les fragments obtenus dans un incubateur à 37 °C pendant au minimum 2 heures.

On ajoute du DMEM au mélange tissu-cocktail enzymatique, et on mélange intimement.

On fait passer le mélange tissu-cocktail enzymatique-DMEM sur un filtre nylon de 40 microns.

On centrifuge pour obtenir un culot de cellules.

On lave les cellules obtenues avec du PBS, et on centrifuge à nouveau.

On élimine les globules rouges, si nécessaire, avec une solution de lyse des globules rouges, on lave au PBS et on centrifuge à nouveau pour obtenir un culot de cellules.

On reprend les cellules du culot dans un petit volume de PBS.

A ce stade, on peut estimer la viabilité des cellules, par coloration au bleu trypan par exemple, et compter les cellules.

On initie ensuite la culture de ces cellules dans des flasques de culture classiques, à 10⁵ cellules par millilitre, dans un milieu qui contient du sérum: milieu de culture RPMI + 10% de sérum de veau foetal (FBS) inactivé à la chaleur;

Le lendemain, on change le milieu de culture pour enlever les cellules non-adhérentes.

Un transfert en milieu sans sérum peut être effectué dès le premier passage, lorsque les cellules sont à 80% de confluence.

L'antibiotique contenu classiquement dans le milieu MEGM est le GA-1000 incluant les antibiotiques gentamicine et amphotéricine-B. Ces antibiotiques peuvent être remplacés notamment par la normocine (Invivogen).

On remplace ensuite le milieu sans sérum environ tous les 3 à 4 jours.

Comme mentionné précédemment, l'invention permet d'obtenir une population cellulaire incluant un pourcentage élevé (jusqu'à plus de 95%) de myofibroblastes et qui poussent dans un milieu qui ne contient pas de sérum.

Les cellules ainsi obtenues présentent notamment les avantages de maintenir leur phénotype en culture et de pousser de manière active sur environ 4 passages.

En outre, ces cellules produisent en abondance du facteur de croissance de l'endothélium vasculaire ou VEGF, le VEGF étant la principale molécule pro-angiogénique.

La figure 1 illustre la production importante de VEGF par les myofibroblastes obtenus avec l'invention.

Elle montre la concentration de VEGF canin dans le surnageant de myofibroblastes au cinquième passage dans le milieu MEGM, au premier jour (colonne de gauche ; partie droite « MYOFIBROBLASTE ») et au deuxième jour (colonne de droite ; partie droite « MYOFIBROBLASTE »). La partie gauche de la figure (« TEMOIN ») sert de témoin : des mesures ont été effectuées dans le milieu de culture seul.

Les mesures ont été réalisées à l'aide d'un kit ELISA VEGF canin (R&D Systems).

Cette figure confirme d'abord que les myofibroblastes sont une source importante de VEGF, la principale molécule pro-angiogénique. Une tumeur a en effet besoin de recruter des vaisseaux sanguins pour s'établir et pousser au-delà d'une certaine taille. Il a été montré que des cellules du stroma sont détournées par la tumeur à cette fin. Par ailleurs, le recrutement de nouveaux vaisseaux sanguins est une étape clé de la réparation tissulaire.

La figure 1 montre en outre que les cellules obtenues par l'invention peuvent être utilisées pour cribler des composés pharmaceutiques à activité anti-angiogénique.

### Liste des références

[1] Orimo et al., Cell 2005, 121: 335-348
[2] Allinen et al., Cancer Cell 2004, 6:17-32
[3] Kinnman et al., Lab Invest 2001, 81:1709-1716
[4] Omary et al., JCI 2007, 117:50-59
[5] Hammond et al., PNAS 1984, 81: 5435-5439
[6] R. Ian Freshney, « Culture of Animal Cells A Manual of Basic Technique », 2005

## Revendications

1. Procédé d'obtention de myofibroblastes, **caractérisé en ce que** on met en culture un échantillon de cellules issu d'une tumeur, de préférence un carcinome dans un milieu de culture sans sérum, ledit milieu étant le MEGM comprenant de l'insuline, de l'hydrocortisone, de l'EGF, un extrait hypophysaire bovin et un antibiotique.

2. Procédé selon la revendication 1 dans lequel une étape (a) comprend:
- une obtention d'une suspension cellulaire à partir d'un échantillon biologique tel qu'un tissu biologique; puis
- une culture initiale des cellules obtenues dans un milieu de culture favorisant la pousse de fibroblastes, par exemple dans un milieu de culture contenant du sérum.

3. Procédé selon l'une quelconque des revendications 1 à 2, dans lequel l'étape (a) comprend:
- une obtention d'une suspension cellulaire à partir d'un échantillon biologique tel qu'un tissu biologique; puis
- une purification de sous-populations cellulaires pour obtenir l'échantillon de cellules incluant essentiellement des fibroblastes.

4. Procédé selon l'une quelconque des revendications 2 à 3, dans lequel l'échantillon biologique contient des myofibroblastes.

5. Culture cellulaire de myofibroblastes obtenue par le procédé tel que décrit dans l'une quelconque des revendications 1 à 4, **caractérisée en ce qu'**au moins 80% des cellules qu'elle contient sont des myofibroblastes.

6. Culture cellulaire selon la revendication 5, qui est dépourvue de sérum.

7. Culture cellulaire selon la revendication 5 ou 6, dans laquelle au moins 95% des cellules qu'elle contient sont des myofibroblastes.

8. Utilisation du procédé tel que décrit dans l'une quelconque des revendications 1 à 4, pour obtenir une culture de cellules dans laquelle les cellules incluent au moins 80% de myofibroblastes dans laquelle les cellules sont dans un milieu sans sérum, ledit milieu étant le MEGM comprenant de l'insuline, de l'hydrocortisone, de l'EGF, un extrait hypophysaire bovin et un antibiotique.

9. Utilisation selon la revendication 8, dans laquelle les cellules incluent au moins 95% de myofibroblastes.

## Patentansprüche

1. Verfahren zur Gewinnung von Myofibroblasten, **dadurch gekennzeichnet, daß** eine Zellprobe aus Zellen, die von einem Tumor stammen, vorzugsweise aus einem Karzinom in einem serumfreien Kulturmedium kultiviert wird, wobei das Medium MEGM ist, das Insulin, Hydrocortison, EGF, ein Rinderhypophysenextrakt und ein Antibiotikum umfasst.

2. Verfahren nach Anspruch 1, wobei Schritt (a) umfasst:
- eine Zellsuspension aus einer biologischen Probe wie beispielsweise einem biologischen Gewebe zu erhalten; dann
- ein anfängliches Züchten der in einem Kulturmedium erhaltenen Zellen, die das Wachstum von Fibroblasten beispielsweise in einem Kulturmedium, das Serum enthält, fördern.

3. Verfahren nach einem der Ansprüche 1 bis 2, wobei Schritt (a) umfasst:
- eine Zellsuspension aus einer biologischen Probe wie beispielsweise einem biologischen Gewebe zu erhalten; dann
- eine Reinigung von Zell-Subpopulationen, um die Probe von Zellen, die hauptsächlich Fibroblasten enthält, zu erhalten.

4. Verfahren nach einem der Ansprüche 2 bis 3, wobei die biologische Probe Myofibroblasten enthält.

5. Zellkultur von Myofibroblasten, die nach einem in den Ansprüchen 1 bis 4 beschriebenen Verfahren erhaltenen werden, **dadurch gekennzeichnet, dass** mindestens 80 % der Zellen Myofibroblasten sind.

6. Zellkultur nach Anspruch 5, die serumfrei ist.

7. Zellkultur nach einem der Ansprüche 5 oder 6, in der mindestens 95 % der Zellen, die sie enthält, Myofibroblasten sind.

8. Verwendung des Verfahrens, das in einem der Ansprüche 1 bis 4 beschrieben ist, um eine Zellkultur zu erhalten, in der die Zellen mindestens 80 % Myofibroblasten enthalten in der die Zellen in einem serumfreien Kulturmedium sind, wobei das Medium MEGM ist, das Insulin, Hydrocortison, EGF, ein Rinderhypophysenextrakt und ein Antibiotikum umfasst.

9. Verwendung nach Anspruch 8, in der die Zellen mindestens 95 % Myofibroblasten enthalten.

## Claims

1. A process for obtaining myofibroblasts, **characterized in that** a sample of cells issued from a tumor, preferably a carcinoma, is cultured in a serum-free culture medium, said medium being the MEGM comprising insulin, hydrocortisone, EGF, a bovine pituitary extract and an antibiotic.

2. The process according to claim 1, wherein step (a) comprises:
- obtaining a cell suspension from a biological sample such as a biological tissue; then
- an initial culturing of the cells obtained in a culture medium favoring the growth of fibroblasts, for example in a culture medium containing serum.

3. The process according to in any one of claims 1 to 2, wherein stage (a) comprises:
- obtaining a cell suspension from a biological sample such as a biological tissue; then
- a purification of cell subpopulations in order to obtain the sample of cells essentially comprising fibroblasts.

4. The process according to any one of claims 2 to 3, wherein the biological sample contains myofibroblasts.

5. A cell culture of myofibroblasts obtained by the process as described in any one of claims 1 to 4, **characterized in that** at least 80% of the cells which it contains are myofibroblasts.

6. The cell culture according to claim 5, which is free of serum.

7. The cell culture according to claim 5 or 6, wherein at least 95% of the cells which it contains are myofibroblasts.

8. The use of the process as described in any one of claims 1 to 4, to obtain a cell culture in which the cells comprise at least 80% of myofibroblasts in which cells are in a serum-free culture medium, said medium being the MEGM comprising insulin, hydrocortisone, EGF, a bovine pituitary extract and an antibiotic.

9. The use according to claim 8, wherein the cells comprise at least 95% of myofibroblasts.
